# EUROPEAN PATENT APPLICATION

(11) **EP 1 671 940 A1**
(43) Date of publication of application: **21.06.2006**
(21) Application number: 04106675.4
(22) Date of filing: 17.12.2004
(51) Int. Cl.: C07C 67/47, C07C 69/587

(54) **Process for the telomerization of conjugated dienes**

(71) Applicant: SHELL INTERNATIONALE RESEARCH MAATSCHAPPIJ B.V., 2596 HR Den Haag (NL)
(72) Inventor: Drent, Eit, 1031 CM Amsterdam (NL); Jager, Willem Wabe, 1031 CM Amsterdam (NL)

(57) **Abstract**

A process for the telomerization of conjugated dienes by contacting the conjugated diene in the presence of a source of platinum with a carboxylic acid or a mixture of carboxylic acids having a dielectric constant, as measured under atmospheric conditions at 20°C, of less than 3.1.

## Description

The present invention provides a process for the telomerization of conjugated dienes, i.e. dienes in which the carbon-carbon double bonds occur in alternation with a single carbon-carbon bond in the carbon atom chain, under catalysis by a platinum catalyst, and in the presence of a carboxylic acid to obtain optionally substituted 2,7-octadienyl esters.

### Background of the invention

Generally, 2,7-octadienyl esters are valuable precursors. For instance, 1-octanol may be conveniently obtained from a 2,7-octadienyl ester by hydrolysis, while 1-octene is accessible from a 2,7-octadienyl ester following hydrogenation and decarboxylation under pyrolysis conditions.

Other alcohols or alkenes are accessible via this route, for instance when 2-methyl-1,3-butadiene (isoprene) is used as a starting material. In these further processes, the exact nature of the ester functionality is of a lesser importance, since it does not recur in the final product.

A process for the preparation of 2,7-octadienyl esters is for instance described in US-A-3,407,224. In this method, several different 2,7-octadienyl esters were obtained in reactions of 1,3-butadiene with a number of carboxylic acids in the presence of a compound of palladium, platinum, or ruthenium and from 1 to 8 moles of a phenolate promoter per mole of the transition metal compound.

The reported yield and selectivity for the respective 2,7-octadienyl esters was however low in spite of the rather large amount of precious metal employed, and rather large amounts of phenolate are required as promoter, which makes the process unsuitable for industrial scale application.

US-A-4,554,375 describes a method for preparing 2,7-octadienyl formate by reacting 1,3-butadiene with formic acid in the presence of a platinum (II) catalyst under high partial pressure of carbon dioxide. Although for this method high turnover numbers are reported, it has a very limited selectivity towards the desired 2,7-octadienyl ester, and mainly yields the non-functionalized 1,3-butadiene dimers 1,6-octadiene and 1,7-octadiene and the telomer 4-vinyl cyclohexene.

Accordingly, there remained the requirement to further improve catalyst activity and the selectivity for the desired 2,7-octadienyl ester products, and more particularly, for the linear 2,7-octadienyl ester products.

It has now been found that the above-identified telomerization process can be very effectively performed when a specific class of carboxylic acids is chosen as co-reactants.

### Summary of the invention

The subject invention thus relates to the a process for the telomerization of conjugated dienes by contacting the conjugated diene in the presence of a source of platinum with a carboxylic acid having a dielectric constant, as measured under atmospheric conditions at 20°C, of less than 3.1.

### Detailed description of the invention

In the process according to the invention, the catalyst system is composed of a suitable source of platinum and an acid reactant serving equally as counterion for the platinum cation. Suitable sources for platinum include platinum metal and complexes and compounds thereof such as platinum salts, for example the salts of platinum and halide acids, nitric acid, sulphuric acid or sulphonic acids; platinum complexes, e.g. with carbon monoxide or acetyl acetonate, or platinum combined with a solid material such as an ion exchanger. Preferably, a salt of platinum and a carboxylic acid is used, suitably a carboxylic acid with up to 12 carbon atoms, such as salts of acetic acid, propionic acid and butanoic acid, since they are readily commercially available, as for instance platinum (II) acetate. Most preferably, though, the counter anion is the conjugated base of the aliphatic acid that also serves as co-reactant. Accordingly, most preferably, a platinum (II) salt of the acid used as reactant is employed, such as for instance platinum (II) hexanoate in the case of hexanoic acid.

The carboxylic acid has a dielectric constant, as measured under atmospheric conditions at 20°C, of less than 3.0. The dielectric constant represents a parameter to measure the polarity of organic molecules. It has been found that acids with a dielectric constant of below 3.0 result in a much higher catalyst activity and selectivity for the desired products. This is also illustrated by the low selectivity of the reactions shown in US-A-4,554,375 and US-A-3,407,224, wherein acids with higher dielectric constants are employed (acetic acid having a dielectric constant of 6.2, formic acid of 58, butyric acid of 3.1). More preferably, the carboxylic acid has a dielectric constant, as measured under atmospheric conditions at 20°C, of less than 2.95, yet more preferably of less than 2.9, again more preferably of less than 2.85, again more preferably of less than 2.7, and most preferably of less than 2.65.

The catalyst system is preferably essentially free from additional polar components, for instance promoters such as phenolates, phosphorus-containing ligands, or polar solvents. "Essentially free" within the context of this specification has the meaning that the reaction mixture comprising the catalyst system contains of less than 1 mole%, calculated on the molar amount of platinum compound serving as the source of platinum, preferably less than 0.1 mole %, again more preferably less than 0.001 mole %, and most preferably less than 0.00001 mole %, calculated on the molar amount of platinum compound serving as the source of platinum. In particular phenolates increase the polarity of the reaction medium strongly, and hence result in a decreased catalyst activity and selectivity. However, the main factor for the catalyst activity appears to reside in the polarity of the acid co-reactant. This is illustrated by the positive effect that the addition of a low polar solvent such a toluene has on the selectivity and catalyst activity in the presence of a highly polar carboxylic acid, as illustrated by comparative example 2 as compared to comparative example 1.

Preferably, the conjugated diene is reacted with a carboxylic acid comprising having at least 5 carbon atoms in the presence of a source of platinum, since these acids have the desired dielectric constant. Preferred are primary carboxylic acids, i.e. carboxylic acids wherein the carbon atom adjacent to the carboxyl group in the carbon-carbon-chain of the acid bears two hydrogen atoms, yet more preferred primary aliphatic or unsaturated carboxyl acids due to their high reactivity as a result of the low steric hindrance. Suitable primary aliphatic carboxylic acids include substituted and unsubstituted aliphatic carboxylic acids comprising at least 5 atoms. Suitable unsaturated acids include fatty acids or mixtures thereof as naturally occurring, and comprise palmitic acid, linoleic acid, oleic acid, and stearic acid. Particularly preferred are primary carboxylic acids carboxylic acids comprising from 5 to 11 carbon atoms in the longest carbon-carbon chain, preferably 6, and yet more preferably more than 6 carbon atoms, and again more preferably 7, and yet more preferably more than 7 carbon atoms, and again more preferably 8 carbon atoms, such as pentanoic acid, 3-methyl pentanoic acid, hexanoic acid, heptanoic acid, 3-methyl heptanoic acid, 4-methyl heptanoic acid, 3-ethyl hexanoic acid, n-octanoic acid, 3,4-dimethyl hexanoic acid, nonanoic acid or decanoic acid, or mixtures thereof. Yet more preferably, the carboxylic acid that acts as a co-reactant comprises more than 8 carbon atoms, yet more preferably 9 or 10 carbon atoms in the carbon-carbon-chain, or mixtures of such acids, since this results in a higher turnover number in the reaction. Again more preferably, the primary carboxylic acid is an unbranched carboxylic acid. Most preferred are n-octanoic acid, n-nonanoic acid and n-decanoic acid, as their low polarity and high pKa was found to increase the reactivity of the catalyst system.

Preferably, the acid has a pKa (measured at 18 °C in water) of more than 4.76, preferably more than 4.80, yet more preferably more than 4.81, and most preferably, the acid has a pKa (measured at 18 °C in water) of more than 4.84.

The subject process may be carried out in the presence of a solvent, preferably of an aprotic solvent such as for instance toluene, yet more preferably in a solvent with a dielectric constant not above the dielectric constant of the acid co-reactant. However, more preferably, the aliphatic acid serves also as sole solvent.

The ratio of moles of acid per mole atom of platinum is not critical. Preferably it ranges from 102:1 to 108:1, more preferably from 102:1 to 106:1. The molar ratio of the conjugated diene to the source of platinum is also not critical. It suitably ranges from 102:1 to 109:1, preferably from 102:1 to 108:1, yet more preferably from 102:1 and 107:1, again preferably between 102:1 and 106:1, yet more preferably between 102:1 and 105:1, and most preferably between 103:1 and 105:1. The quantity in which the complete catalyst system is used is not critical and may vary within wide limits, however preferably, the catalyst is present in an amount below 0.01% mole based on the total of reactants.

In the subject process, 2 moles of conjugated dienes are reacted with 1 mole of an aliphatic carboxylic acid. In order to ensure high conversion, preferably at least 2 moles of the conjugated diene area present per mole of carboxylic acid. Since many conjugated dienes have a lower boiling point than the acid employed, it is often easier to remove a surplus of diene once the acid has been consumed. Therefore, preferably the molar ratio of amount of diene to acid is in the range of from 2 to 100, yet more preferably in the range of from 2.2 to 10. The process may be conducted batch-wise, a semi-continuous operation or in a continuous operation. The ratio (v/v) of diene and aliphatic acid in the feed in the case of a continuous process operation can vary between wide limits and suitably lies in the range of 1:0.1 to 1:10.

The conjugated diene reactant has at least 4 carbon atoms. Preferably the diene has from 4 to 20 and more preferably from 4 to 14 carbon atoms. However, in a different preferred embodiment, the process may also be applied to molecules that contain conjugated double bonds within their molecular structure, for instance within the chain of a polymer such as a synthetic rubber. The conjugated diene can be substituted or non -substituted. Preferably the conjugated diene is a non-substituted diene. Examples of useful conjugated dienes are the 1,3-butadiene, conjugated pentadienes, conjugated hexadienes, cyclopentadiene and cyclohexadiene, all of which may be substituted. Of particular commercial interest are 1,3-butadiene and 2-methyl-1,3-butadiene (isoprene). The feed containing the diene reactant does not necessarily have to be free of admixture with alkanes. The subject invention also relates to the novel products obtainable by the subject process, in particular to the 2,7-octadienyl esters or the mixture thereof obtainable, in particular the esters obtained from n-hexanoic, n-heptanoic, n-octanoic acid, n-nonanoic acid and/or n-decanoic acid, or from fatty acids as set out above, or from mixtures thereof, and more in particular from 1,3-butadiene or isoprene.

The telomerization reaction is conveniently carried out at moderate temperatures and pressures. Suitable reaction temperatures are in the range of 0-250°C, more preferably in the range of 50-200°C, yet more preferably in the range of from 80-150°C. The reaction pressure is usually at least atmospheric. Suitable pressures are in the range of 0.1 to 15 MPa (1 to 150 bar), preferably in the range of 0.5 to 10 MPa (5 to 100 bar), yet again preferably in the range of 0.6 to 8.5 MPa (5 to 85 bar). Higher pressures require special equipment provisions.

The invention will be illustrated by the following non-limiting examples.

### Example 1: Telomerization of 1,3-butadiene with hexanoic acid

A 350 ml magnetically stirred autoclave, made of HASTELLOY C (HASTELLOY C is a trademark), was successively charged with 40 ml n-hexanoic acid (having a dielectric constant, as measured under atmospheric conditions at 20°C, of 2.6) and 0.1 mmol platinum (II) 2,4-pentanedionate. The autoclave was flushed with nitrogen, then 30 ml 1,3-butadiene were pumped into the autoclave. The autoclave was sealed, heated to 100 °C and maintained at that temperature for 10 hours. Finally the autoclave was cooled and the reaction mixture was analyzed with HPLC.

The average reaction rate (mol per mol Pt per hour) of this batch operation is defined as the mean rate of butadiene consumption over acid consumption.

Butadiene conversion was determined as 80%, the selectivity towards the 2,7-octadienyl ester was 95%, which corresponded to an average reaction rate of 200 mol/mol Pt/hour. The obtained product had a linearity of 92%. The byproducts consisted mainly in 4-vinyl cyclohexene.

### Example 2: Telomerization of 1,3-butadiene with nonanoic acid

Example 1 was repeated, however employing n-nonanoic acid (having a dielectric constant, as measured under atmospheric conditions at 20°C, of 2.3) instead of hexanoic acid. Butadiene conversion was determined as 80%, the selectivity towards the 2,7-octadienyl ester was 95%, which corresponded to an average reaction rate of 250 mol/mol Pt/hour. The obtained product had a linearity of 91%. The byproducts consisted mainly in 4-vinyl cyclohexene.

### Comparative Example 1: Telomerization of 1,3-butadiene with acetic acid

Example 1 was repeated, however employing 40 ml of acetic acid (having a dielectric constant, as measured under atmospheric conditions at 20°C, of 6.2) instead of hexanoic acid. Butadiene conversion was determined as less than 10%, which corresponded to an average reaction rate of only 10 mol/mol Pt/hour, and the obtained product had a linearity of 75%.

### Comparative Example 2: Telomerization of 1,3-butadiene with acetic acid in toluene

Comparative Example 1 was repeated, however employing 10 ml of acetic acid and 40 ml of toluene. Butadiene average reaction rate was increased to 60 mol/mol Pt/hour, and the obtained product had a linearity of 87%.

From the examples, it is clearly visible that carboxylic acids having a dielectric constant, as measured under atmospheric conditions at 20°C, of below 3.1 yield substantially higher amounts of the desired linear ester telomerization products than acids having a higher polarity, such as for instance acetic acid.

## Claims

1. A process for the telomerization of conjugated dienes by contacting the conjugated diene in the presence of a source of platinum with a carboxylic acid or a mixture of carboxylic acids having a dielectric constant, as measured under atmospheric conditions at 20°C, of less than 3.1.

2. A process according to claim 1, wherein the carboxylic acid has a pKa (measured at 18 °C in water) of more than 4.76.

3. A process according to claim 1 or claim 2, wherein the carboxylic acid is a primary aliphatic carboxylic acid comprising at least 5 carbon atoms or mixtures thereof.

4. A process according to any one of claims 1 to 3, wherein the carboxylic acid has from 5 to 16 carbon atoms in the longest carbon-carbon-chain or mixtures thereof.

5. A process according to any one of claims 1 to 4, wherein the primary aliphatic carboxylic acid is selected from n-hexanoic, n-heptanoic, n-octanoic acid, n-nonanoic acid and/or n-decanoic acid, or mixtures thereof.

6. A process according to any one of claims 1 to 4, wherein the acid is a fatty acid, or mixtures thereof.

7. A process according to any one of claims 1 to 6, wherein the process is carried out in the presence of an aprotic solvent.

8. A process according to any one of claims 1 to 7, wherein the conjugated diene is 1,3-butadiene or 2-methyl-1,3-butadiene.

9. The 2,7-octadienyl ester or the mixture of esters obtainable by the process according to claim 5 or claim 6.

10. The 2,7-octadienyl ester of n-hexanoic acid or n-nonanoic acid.
